# EUROPÄISCHE PATENTSCHRIFT

(11) **EP 0 763 516 B1**
(45) Veröffentlichungstag und Bekanntmachung des Hinweises auf die Patenterteilung: **20.06.2001**
(21) Anmeldenummer: 96114418.5
(22) Anmeldetag: 10.09.1996
(51) Int. Cl.: C07C 59/115, C07C 69/675, C07C 51/487, C07C 67/333

(54) **Herstellung und Verwendung der reinen Enantiomere der 8-Halogen-6-hydroxyoctansäuren, ihrer Alkylester und ihrer Salze mit reinen Enantiomeren des alpha-Methylbenzylamins**
Preparation and utilisation of pure enantiomers of 8-halogen-6-hydroxyoctanoic acid, their alkyl esters and their salts with pure enantiomers of alpha-methyl-benzylamines
Préparation et utilisation des énantiomères purs des acides 8-halogène-6-hydroxyoctanoiques, leurs esters alkyliques et leurs sels avec les énantiomères purs de l'alpha-méthylbenzylamine

(30) Priorität: 13.09.1995 DE 19533882
(43) Veröffentlichungstag der Anmeldung: 19.03.1997
(73) Patentinhaber: Arzneimittelwerk Dresden GmbH, 01445 Radebeul (DE)
(72) Erfinder: Gewald, Rainer, Dr., 01217 Dresden (DE); Laban, Gunter, Dr., 01465 Langebrück (DE); Beisswenger, Thomas, Dr., 01445 Radebeul (DE)
(74) Vertreter: Wibbelmann, Jobst, Dr., Dipl.-Chem.

(56) Entgegenhaltungen:
- DE-A- 4 137 773
- US-A- 2 975 198
- US-A- 2 980 716

## Beschreibung

Die Erfindung betrifft neue (+)- und (-)-8-Halogen-6-hydroxyoctansäuren (Halogen = Chlor, Brom und Jod) der Formel I, deren Alkylester der Formel II und deren Salze mit optisch aktivem α-Methylbenzylamin der Formel III als Zwischenprodukte zur Herstellung der enantiomerenreinen α-Liponsäuren der Formel IV sowie der enantiomerenreinen Dihydroliponsäuren der Formel V. α-Liponsäure ist 1,2-Dithiolan-3-pentansäure (Thioctsäure).

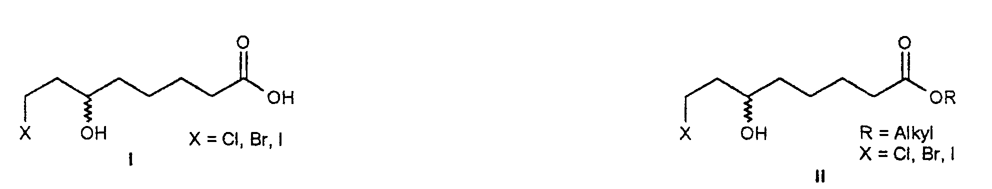

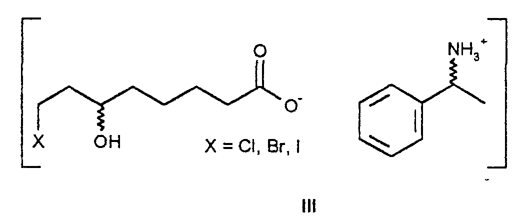

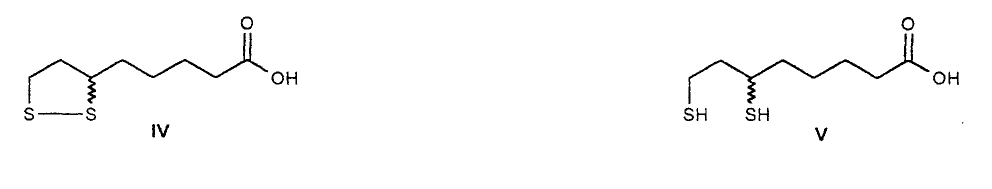

Das R-Enantiomer der α-Liponsäure ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z.B. Brenztraubensäure) ist α-Liponsäure von essentieller Bedeutung. Das Razemat der α-Liponsäure ist pharmakologisch wirksam und weist antiphlogistische und antinociceptive (analgetische) sowie zytoprotektive Eigenschaften auf. Eine wichtige medizinische Indikation ist die Behandlung der diabetischen Polyneuropathie. Nach neueren Ergebnissen (vgl. CA 116: 207360) kann α-Liponsäure möglicherweise Bedeutung bei der Bekämpfung durch HIV-1- und HTLV IIIB-Viren bedingter Krankheiten erlangen.

Bei den reinen optischen Isomeren der α-Liponsäure (R- und S-Form, d.h. R-α-Liponsäure und S-α-Liponsäure) ist im Unterschied zu dem Razemat das R-Enantiomer vorwiegend antiphlogistisch und das S-Enantiomer vorwiegend antinociceptiv wirksam (EP 0427247, 08.11.90). Daher ist die Synthese der reinen Enantiomere von großer Wichtigkeit.

Bekannte Herstellungsverfahren der enantiomerenreinen α-Liponsäure umfassen die Razematspaltung der α-Liponsäure oder ihrer Vorstufen, asymmetrische Synthesen unter Einsatz chiraler Auxilarien , "chiral pool"-Synthesen unter Verwendung von in der Natur vorkommenden optisch aktiven Ausgangsverbindungen sowie mikrobielle Synthesen (Übersichtsartikel: J.S. Yadav et al., J. Sci. Ind. Res. 1990, 49, 400; sowie: E. Walton et al. J. Am. Chem. Soc. 1955, 77, 5144; D.S. Acker und W.J. Wayne, J. Am. Chem. Soc. 1957, 79, 6483; L.G. Chebotareva und A.M. Yurkevich, Khim.-Farm. Zh. 1980, 14, 92; A.G. Tolstikov et al., Bioorg. Khim. 1990, 16, 1670; L. Dasaradhi et al., J. Chem. Soc., Chem. Commun. 1990, 729; A.S. Gopalan et al., J. Chem. Perkin Trans. 1 1990, 1897; A.S. Gopalan et al., Tetrahedron Lett. 1989, 5705; EP 0487986 A2, 14.11.91). In dem US-Patent 2,792,406 werden als mögliche racemische Vorstufen die D,L-8-Chlor-6-hydroxy-octansäure und deren Alkylester genannt. Als potentielle optisch aktive Reagentien zur Racematspaltung werden dort Brucin, Strychnin, Cinchodin, L-Menthoxy-acetylchlorid, L-Menthylisocynat, sowie D- und L-Camphersulfonylchlorid angegeben.

Davon stellt die Razematspaltung über die Bildung von diastereomeren Salzen der α-Liponsäure mit optisch aktivem α-Methylbenzylamin ( DE-OS 4137773.7, 16.11.91) die bisher wirtschaftlichste Variante dar. Der Nachteil dieses Verfahrens besteht jedoch darin, daß die Razemattrennung erst auf der letzten Stufe der Synthesesequenz erfolgt und das unerwünschte Enantiomer der α-Liponsäure weder razemisiert noch invertiert werden kann. Auch bei den anderen bekannten Razematspaltungsverfahren auf Vorstufen der α-Liponsäure kann jeweils nur ein Enantiomer in das gewünschte optische Isomer der α-Liponsäure überführt und somit nur eine theoretische Ausbeute von 50% erreicht werden (E. Walton et al. J. Am. Chem. Soc. 1955, 77, 5144; D.S. Acker und W.J. Wayne, J. Am. Chem. Soc. 1957, 79, 6483; L.G. Chebotareva und A.M. Yurkevich, Khim.-Farm. Zh. 1980, 14, 92).

Aufgabe der Erfindung ist es deshalb, Zwischenprodukte für die Herstellung der enatiomerenreinen α-Liponsäuren und Dihydroliponsäuren bereitzustellen, mit deren Hilfe ein Enantiomer der α-Liponsäure oder Dihydroliponsäure mit einer theoretischen Ausbeute von 100 % herstellbar ist..

Die Ausgangsverbindungen zur Herstellung der Zwischenprodukte, die razemische 8-Chlor-6-hydroxy-octansäure und die razemische 8-Brom-6-hydroxy-octansäure der Formel I (X= Cl, Br), werden auf bekanntem Wege durch Hydrolyse ihrer razemischen Alkylester der Formel II (X= Cl, Br) gewonnen (Y. Deguchi und K. Nakanishi, Yakugaku Zasshi 1963, 83, 701). Die razemische 8-Jod-6-hydroxy-octansäure der Formel I (X= I) wird durch Umsetzung der razemischen 8-Chlor-6-hydroxy-octansäure der Formel I (X= Cl) mit Natriumjodid in Aceton in hohen Ausbeuten hergestellt.

Bei der Herstellung von Salzen der Formel III der reinen optischen Isomeren der 8-Halogen-6-hydroxy-octansäuren mit den reinen optischen Isomeren des α-Methylbenzylamins wird so vorgegangen, daß in einem geeigneten Lösemittel die Isomere bei erhöhter Temperatur, beispielsweise bei 30°C bis 100°C, insbesondere bei 40 bis 60°, aufgelöst werden und durch Kristallisation bei 10°C bis 30°C, insbesondere bei 20°C die reinen diastereomeren Salze isoliert werden. Als Lösemittel kommen neben Wasser aliphatische Kohlenwasserstoffe mit einer Kohlenstoffkettenlänge zwischen 3 und 10 Kohlenstoffatomen, aromatische Kohlenwasserstoffe, die flüssig sind, Ester aus aliphatischen oder cycloaliphatischen Carbonsäuren mit 2 bis 6 Kohlenstoffatomen und aliphatischen oder cycloaliphatischen Alkoholen mit 2 bis 6 Kohlenstoffatomen, aliphatischen oder cycloaliphatischen Alkoholen mit 1 bis 6 Kohlenstoffatomen, Ether und Glycolether oder homogene Gemische der genannten Lösemittel in Frage. Besonders bevorzugte Lösemittel sind Essigsäureethylester, Cyclohexan, Toluol und deren homogene Gemische.

Überraschenderweise zeigen die diastereomeren Salze erhebliche Löslichkeitsunterschiede, so daß bei einer Umsetzung des Razemats der 8-Halogen-6-hydroxy-octansäuren der Formel I mit einem optisch reinen Isomer des α-Methylbenzylamins selektiv ein diastereomeres Salz der Formel III bevorzugt isoliert wird. Besonders vorteilhaft ist es, zu den razemischen 8-Halogen-6-hydroxy-octansäure-Lösungen nur 0,3 bis 0,8, vorzugsweise jedoch 0,5 bis 0,6 des molaren Äquivalents eines reinen Enantiomers des α-Methylbenzylamins zuzusetzen. Dabei kann selektiv ein diastereomeres Salz bevorzugt isoliert werden. Das in der Mutterlauge jetzt im Überschuß vorhandene Enantiomer der entsprechenden 8-Halogen-6-hydroxy-octansäure kann durch Zusatz des anderen Enantiomers des α-Methylbenzylamins besonders angereichert werden. Diese Vorgehensweise eignet sich für ein kontinuierliches Herstellverfahren von sowohl (+)-8-Halogen-6-hydroxy-octansäuren als auch (-)-8-Halogen-6-hydroxy-octansäuren, wobei die beiden reinen Enantiomere weitgehend verlustfrei erhalten werden können. Durch Umkristallisation aus den reinen, bereits genannten Lösemitteln oder deren homogenen Mischungen können diese diastereomeren Salze aufgereinigt werden, so daß schließlich reine Salze vorliegen.

Die bei den obengenannten Trennungsschritten erhaltenen reinen Salze der Formel III aus den (+)-8-Halogen-6-hydroxy-octansäuren und R-(+)-α-Methylbenzylamin beziehungsweise (-)-8-Halogen-6-hydroxyoctansäuren und S-(-)-α-Methylbenzylamin können durch Zusatz von Säuren, z.B. Mineralsäuren, oder Basen, z.B. Alkalihydroxyden, gespalten werden und die reinen (+)-8-Halogen-6-hydroxy-octansäuren oder die reinen (-)-8-Halogen-6-hydroxy-octansäuren extraktiv isoliert werden.

Die Enantiomere der 8-Halogen-6-hydroxy-octansäuren der Formel I lassen sich erfindungsgemäß stereospezifisch unter Erhalt der Konfiguration in Gegenwart von katalytischen Mengen HCI in ihre Alkylester der Formel II, vorzugsweise Methylester, umwandeln. Die Umsetzung wird bei 50 bis 100°C, insbesondere bei 60°C, im entsprechenden Alkohol als Lösungsmittel vorgenommen.

Die Reinheit der optischen Isomere und der diastereomeren Salze wurde mittels der spezifischen optischen Drehwerte bestimmt. Weiterhin wurden die relativen Gehalte der optischen Isomere der 8-Halogen-6-hydroxy-octansäuren der Formel I und der α-Liponsäure der Formel IV durch HPLC an optisch aktiven Säulen mit einer Nachweisgrenze von 0,5% ermittelt. Zusätzlich erfolgte die Bestimmung der optischen Reinheit der 8-Halogen-6-hydroxy-octansäurealkylester der Formel II durch ¹H-NMR-Analyse der diastereomeren Ester, die bei der Reaktion mit (S)-(+)-O-Acetylmandelsäure gebildet werden.

Die vorliegende Erfindung ermöglicht es, die (+)- und (-)-8-Halogen-6-hydroxy-octansäuren (Halogen = Chlor, Brom und Jod) der Formel I, deren Alkylester der Formel II und deren Salze mit optisch aktivem α-Methylbenzylamin der Formel III als Zwischenprodukte zur Herstellung der enantiomerenreinen α-Liponsäuren der Formel IV sowie der enantiomerenreinen Dihydroliponsäuren der Formel V.auf einfache und wirtschaftliche Weise in hoher chemischer und optischer Ausbeute zugänglich zu machen.

Als Beispiel für die Herstellung enantiomerenreiner α-Liponsäure oder Dihydroliponsäure aus den Zwischenprodukten der vorliegenden Erfindung wird im folgenden Schema die Herstellung der R(+)-α-Liponsäure gezeigt. Die einzelnen Umsetzungen können mittels an sich für das jeweilige Razemat bekannter Verfahren durchgeführt werden.

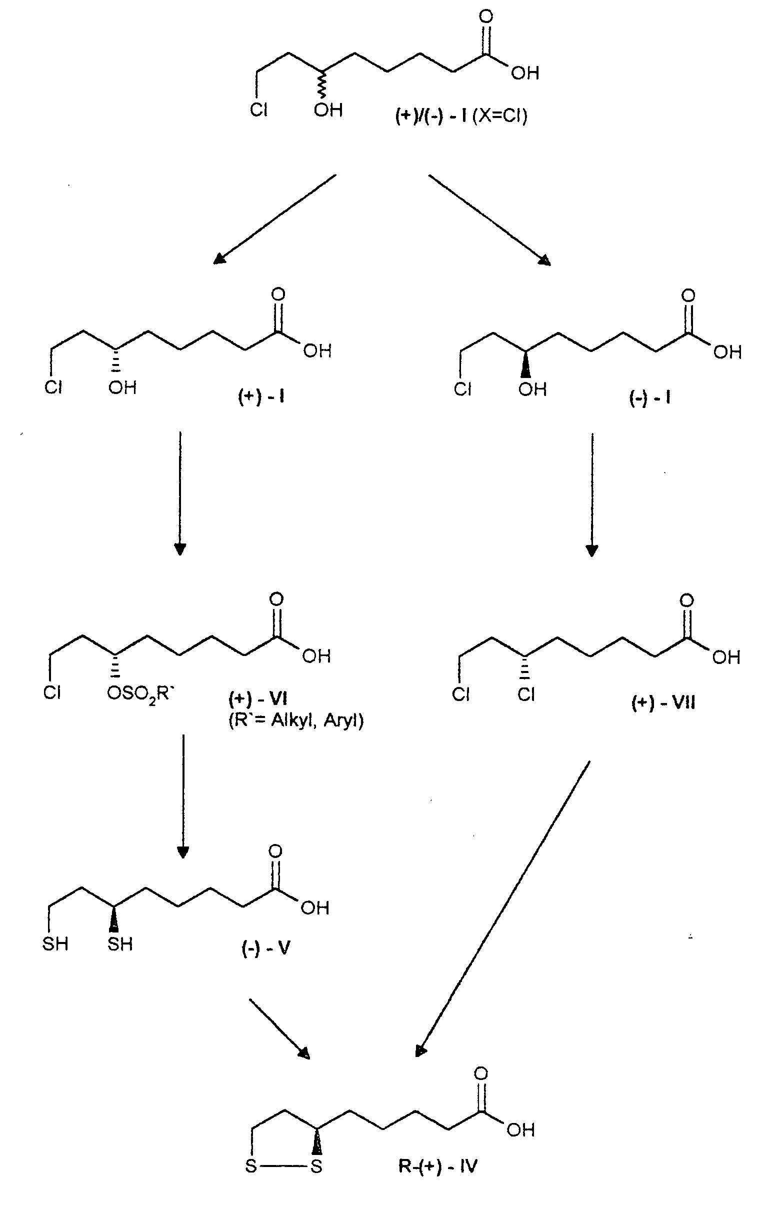

In analoger Weise ist die S(-)-Liponsäure zugänglich, indem für den Verfahrensablauf auf der linken Seite des Schemas die (-)-8-Chlor-6-hydroxyoctansäure verwendet wird, während die (+)-8-Chlor-6-hydroxy-octansäure auf der rechten Seite eingesetzt wird. Die enantiomerenreinen Dihydroliponsäuren können dann durch Reduktion der entsprechenden enatiomerenreinen α-Liponsäuren mittels bekannter Verfahren hergestellt werden.

Die Erfindung wird durch nachfolgende Beispiele näher erläutert.

### Beispiel 1

1,94 g (10 mmol) (+)-8-Chlor-6-hydroxy-octansäure (+)-I (X=CI) wurden bei 60°C in 30 ml eines Gemisches aus Ethylacetat und Cyclohexan (1:1) aufgelöst. Innerhalb von 5 min wurden 1,21 g (10 mmol) R-(+)-α-Methylbenzylamin zudosiert. Über einen Zeitraum von 1 h wurde auf 20°C abgekühlt. Der Niederschlag wurde abfiltriert und zweimal mit 3 ml eines Gemisches aus Ethylacetat und Cyclohexan (1:1) nachgewaschen. Das Salz wurde im Vakuum bei 40°C getrocknet.

Man erhielt 3,12 g (99% d. Th.) (+)-8-Chlor-6-hydroxy-octansäure- R-(+)-α-methylbenzylamin-Salz (+)/(+)-III (X=Cl), [α]_{D}²⁰ = +22,7° (c = 1; Ethanol), e.e.: >99% (HPLC), Löslichkeit im Gemisch Ethylacetat/Cyclohexan (1:1) 0,09% (20°C), Löslichkeit im Gemisch Ethylacetat/Cyclohexan (3:1) 0,16% (20°C), Schmelzpunkt 122-124°C.

### Beispiel 2

1,94 g (10 mmol) (-)-8-Chlor-6-hydroxy-octansäure (-)-I (X=Cl) wurden bei 60°C in 30 ml eines Gemisches aus Ethylacetat und Cyclohexan (1:1) aufgelöst. Innerhalb von 5 min wurden 1,21 g (10 mmol) R-(+)-α-Methylbenzylamin zudosiert. Über einen Zeitraum von 1 h wurde auf 20°C abgekühlt. Der Niederschlag wurde abfiltriert und zweimal mit 3 ml eines Gemisches aus Ethylacetat und Cyclohexan (1:1) nachgewaschen. Das Salz wurde im Vakuum bei 40°C getrocknet.

Man erhielt 3,05 g (97% d. Th.) (-)-8-Chlor-6-hydroxy-octansäure - R-(+)-α-methylbenzylamin-Salz (-)/(+)-III (X=Cl), [α]_{D}²⁰ = +10,3° (c = 1; Ethanol), e.e.: >99% (HPLC), Löslichkeit im Gemisch Ethylacetat/Cyclohexan (1:1) 0,30% (20°C), Löslichkeit im Gemisch Ethylacetat/Cyclohexan (3:1) 0,54% (20°C), Schmelzpunkt 94-95°C.

### Beispiel 3

1,94 g (10 mmol) (-)-8-Chlor-6-hydroxy-octansäure (-)-I (X=Cl) wurden bei 60°C in 30 ml eines Gemisches aus Ethylacetat und Cyclohexan (1:1) aufgelöst. Innerhalb von 5 min wurden 1,21 g (10 mmol) S-(-)-α-Methylbenzylamin zudosiert und, wie in Beispiel 1 beschrieben, aufgearbeitet.

Man erhielt 3,11 g (99% d. Th.) (-)-8-Chlor-6-hydroxy-octansäure- S-(-)-α-methylbenzylamin-Salz (-)/(-)-III (X=Cl), [α]_{D}²⁰ = -22,7° (c = 1; Ethanol), e.e.: >99% (HPLC), Löslichkeit im Gemisch Ethylacetat/Cyclohexan (1:1) 0,09% (20°C), Löslichkeit im Gemisch Ethylacetat/Cyclohexan (3:1) 0,16% (20°C), Schmelzpunkt 122-124°C.

### Beispiel 4

1,94 g (10 mmol) (+)-8-Chlor-6-hydroxy-octansäure (+)-I (X=Cl) wurden bei 60°C in 30 ml eines Gemisches aus Ethylacetat und Cyclohexan (1:1) aufgelöst. Innerhalb von 5 min wurden 1,21 g (10 mmol) S-(-)-α-Methylbenzylamin zudosiert und, wie in Beispiel 1 beschrieben, aufgearbeitet.

Man erhielt 3,04 g (97% d. Th.) (+)-8-Chlor-6-hydroxy-octansäure- S-(-)-α-methylbenzylamin-Salz (+)/(-)-III (X=Cl), [α]_{D}²⁰ = -10,3° (c = 1; Ethanol), e.e.: >99% (HPLC), Löslichkeit im Gemisch Ethylacetat/Cyclohexan (1:1) 0,30% (20°C), Löslichkeit im Gemisch Ethylacetat/Cyclohexan (3:1) 0,54% (20°C), Schmelzpunkt 94-95°C.

### Beispiel 5

39,9 g (204 mmol) razemische 8-Chlor-6-hydroxy-octansäure (+)/(-)-I (X=CI) wurden bei 40°C in 155 ml eines Gemisches aus Ethylacetat und Cyclohexan (1:1) aufge-löst. Innerhalb von 10 min wurden 13,5 g (112 mmol) R-(+)-α-Methylbenzylamin zudosiert. Anschließend wurde über einen Zeitraum von 2 h auf 20°C abgekühlt, filtriert und der Niederschlag mit 20 ml Lösemittelgemisch Ethylacetat/Cyclohexan (1:1) und 30 ml Cyclohexan nachgewaschen. Das Salz wurde zweimal aus 400 ml Ethylacetat/Cyclohexan (3:1) umkristallisiert und im Vakuum bei 40°C getrocknet. Man erhielt 20,5 g (+)-(+)-Diastereomerensalz, α = +22,7° (c = 1; Ethanol).

Das Salz wurde bei 20°C in 220 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 20 ml 2 N HCI und zweimal mit 20 ml Wasser gewaschen und über Magnesiumsulfat getrocknet. Nach Abziehen des Lösemittels im Vakuum wurden 10,8 g (54% d. Th.) (+)-8-Chlor-6-hydroxy-octansäure (+)-I (X=CI) erhalten; [α]_{D}²⁰ = +24,5° (c = 1; Ethanol), e.e.: >99% (HPLC), Schmelzpunkt 29-30°C.

### Beispiel 6

33,9 g (173 mmol) razemische 8-Chlor-6-hydroxy-octansäure (+)/(-)-I (X=CI) wurden bei 40°C in 130 ml eines Gemisches aus Ethylacetat und Cyclohexan (1:1) aufgelöst. Innerhalb von 10 min wurden 11,5 g (95 mmol) S-(-)-α-Methylbenzylamin zudosiert. Anschließend wurde über einen Zeitraum von 2 h auf 20°C abgekühlt, filtriert und der Niederschlag mit 17 ml Lösemittelgemisch Ethylacetat/Cyclohexan (1:1) und 25 ml Cyclohexan nachgewaschen. Das Salz wurde zweimal aus 340 ml Ethylacetat/Cyclohexan (3:1) umkristallisiert und im Vakuum bei 40°C getrocknet. Man erhielt 17,2 g (-)-(-)-Diastereomerensalz, α = -22,7° (c = 1; Ethanol).

Das Salz wurde bei 20°C in 190 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 17 ml 2 N HCl und zweimal mit 20 ml Wasser gewaschen und über Magnesiumsulfat getrocknet. Nach Abziehen des Lösemittels im Vakuum wurden 9,1 g (53% d. Th.) (-)-8-Chlor-6-hydroxy-octansäure (-)-I (X=Cl) erhalten;[α]_{D}²⁰ = -24,5° (c = 1; Ethanol), e.e.: >99% (HPLC), Schmelzpunkt 29-30°C.

### Beispiel 7

6,4 g (32,9 mmol) (+)-8-Chlor-6-hydroxy-octansäure (+)-I (X=CI) wurden in 100 ml absolutem Methanol nach Zugabe von 0,4 ml konzentrierter Salzsäure 2 h am Rückfluß erhitzt. Danach wurde das Lösemittel im Vakuum abgezogen. Man erhielt 6,6 g (97% d. Th.) (+)-8-Chlor-6-hydroxy-octansäuremethylester (+)-II (X=CI, R=Me), [α]_{D}²⁰ = +24,5° (c = 1; Ethanol), e.e.: >99% (1 H NMR).

### Beispiel 8

7,7 g (39,5 mmol) (-)-8-Chlor-6-hydroxy-octansäure (-)-I (X=Cl) wurden in 120 ml absolutem Methanol nach Zugabe von 0,5 ml konzentrierter Salzsäure 2 h am Rückfluß erhitzt. Danach wurde das Lösemittel im Vakuum abgezogen. Man erhielt 7,9 g (97% d. Th.) (-)-8-Chlor-6-hydroxy-octansäuremethylester (-)-II (X=CI, R=Me), [α]_{D}²⁰ = -24,5° (c = 1; Ethanol), e.e.: >99% (¹H NMR).

### Beispiel 9

9,0 g (46,3 mmol) razemische 8-Chlor-6-hydroxy-octansäure I (X=CI) und 10,6 g (70,0 mmol) Natriumjodid wurden in 100 ml Aceton gelöst und 12 Stunden am Rückfluß erhitzt. Der ausgefallene Niederschlag wurde abgesaugt, mit 10 ml Aceton gewaschen und das Filtrat auf 30 ml eingeengt. Dann wurden 100 ml Diethylether zugegeben, mit 10 ml Wasser gewaschen, über Natriumsulfat getrocknet und das Lösungsmittel im Vakuum abgezogen. Man erhielt 12,1 g (91% d. Th.) razemische 8-Jod-6-hydroxy-octansäure I (X=I).

### Beispiel 10

2,85 g (10 mmol) (+)-8-Jod-6-hydroxy-octansäure (+)-I (X=I) wurden bei 60°C in 30 ml eines Gemisches aus Ethylacetat und Cyclohexan (1:1) aufgelöst. Innerhalb von 5 min wurden 1,21 g (10 mmol) R-(+)-α-Methylbenzylamin zudosiert. Über einen Zeitraum von 1 h wurde auf 20°C abgekühlt. Der Niederschlag wurde abfiltriert und zweimal mit 3 ml eines Gemisches aus Ethylacetat und Cyclohexan (1:1) nachgewaschen. Das Salz wurde im Vakuum bei 40°C getrocknet.

Man erhielt 4,01 g (99% d. Th.) (+)-8-Jod-6-hydroxy-octansäure- R-(+)-α-methylbenzylamin-Salz (+)/(+)-III (X=I), [α]_{D}²⁰ = +23,6° (c = 1; Ethanol), e.e.: >99% (HPLC), Löslichkeit im Gemisch Ethylacetat/Cyclohexan (1:1) 0,07% (20°C), Schmelzpunkt 108-111°C.

### Beispiel 11

2,85 g (10 mmol) (-)-8-Jod-6-hydroxy-octansäure (-)-I (X=I) wurden bei 60°C in 30 ml eines Gemisches aus Ethylacetat und Cyclohexan (1:1) aufgelöst. Innerhalb von 5 min wurden 1,21 g (10 mmol) R-(+)-α-Methylbenzylamin zudosiert. Über einen Zeitraum von 1 h wurde auf 20°C abgekühlt. Der Niederschlag wurde abfiltriert und zweimal mit 3 ml eines Gemisches aus Ethylacetat und Cyclohexan (1:1) nachgewaschen. Das Salz wurde im Vakuum bei 40°C getrocknet.

Man erhielt 3,93 g (97% d. Th.) (-)-8-Jod-6-hydroxyoctansäure- R-(+)-α-methylbenzylamin-Salz (-)/(+)-III (X=I), [α]_{D}²⁰ = -14,2° (c = 1; Ethanol), e.e.: >99% (HPLC), Löslichkeit im Gemisch Ethylacetat/Cyclohexan (1:1) 0,50% (20°C), Schmelzpunkt 84-86°C.

### Beispiel 12

2,85 g (10 mmol) (-)-8-Jod-6-hydroxy-octansäure (-)-I (X=I) wurden bei 60°C in 30 ml eines Gemisches aus Ethylacetat und Cyclohexan (1:1) aufgelöst. Innerhalb von 5 min wurden 1,21 g (10 mmol) S-(-)-α-Methylbenzylamin zudosiert und, wie in Beispiel 10 beschrieben, aufgearbeitet.

Man erhielt 4,02 g (99% d. Th.) (-)-8-Jod-6-hydroxy-octansäure - S-(-)-α-methylbenzylamin-Salz (-)/(-)-III (X=I), [α]_{D}²⁰ = -23,6° (c = 1; Ethanol), e.e.: >99% (HPLC), Löslichkeit im Gemisch Ethylacetat/Cyclohexan (1:1) 0,07% (20°C), Schmelzpunkt 108-111°C.

### Beispiel 13

2,85 g (10 mmol) (+)-8-Jod-6-hydroxy-octansäure (+)-I (X=I) wurden bei 60°C in 30 ml eines Gemisches aus Ethylacetat und Cyclohexan (1:1) aufgelöst. Innerhalb von 5 min wurden 1,21 g (10 mmol) S-(-)-α-Methylbenzylamin zudosiert und, wie in Beispiel 10 beschrieben, aufgearbeitet.

Man erhielt 3,90 g (96% d. Th.) (+)-8-Jod-6-hydroxy-octansäure- S-(-)-α-methylbenzylamin-Salz (+)/(-)-III (X=I), [α]_{D}²⁰ = +14,2° (c = 1; Ethanol), e.e.: >99% (HPLC), Löslichkeit im Gemisch Ethylacetat/Cyclohexan (1:1) 0,50% (20°C), Schmelzpunkt 84-86°C.

### Beispiel 14

8,1 g (+)-8-Jod-6-hydroxy-octansäure-R-(+)-α-methylbenzylamin-Salz (+)/(+)-III (X=I) wurden bei 20°C in 90 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 10 ml 2 N HCI und zweimal mit 10 ml Wasser gewaschen und über Magnesiumsulfat getrocknet. Nach Abziehen des Lösungsmittels im Vakuum wurden 5,2 g (90% d. Th.) (+)-8-Jod-6-hydroxyoctansäure (+)-I (X=I) erhalten; [α]_{D}²⁰ = +24,7° (c = 1; Ethanol), e.e.: >99%.

### Beispiel 15

8,1 g (-)-8-Jod-6-hydroxy-octansäure - S-(-)-α-methylbenzylamin-Salz (-)/(-)-III (X=I) wurden bei 20°C in 90 ml Diethylether suspendiert. Unter Eiskühlung und Rühren wurde mit 3 N Salzsäure langsam auf einen pH-Wert von 1 eingestellt, wobei das Salz in Lösung geht. Nach weiteren 30 min wurden die Phasen getrennt und die organische Phase einmal mit 10 ml 2 N HCI und zweimal mit 10 ml Wasser gewaschen und über Magnesiumsulfat getrocknet. Nach Abziehen des Lösungsmittels im Vakuum wurden 5,1 g (89% d. Th.) (-)-8-Jod-6-hydroxy-octansäure (-)-I (X=I) erhalten; [α]_{D}²⁰ = -24,7° (c = 1; Ethanol), e.e.: >99%.

## Patentansprüche

1. (+)- und (-)-8-Halogen-6-hydroxy-octansäure, wobei Halogen Chlor, Brom oder Jod bedeutet.

2. (+)- und (-)-8-Halogen-6-hydroxy-octansäurealkylester, wobei Halogen Chlor, Brom oder Jod und Alkyl eine geradkettige oder verzweigte C₁ - C₄-Alkylgruppe bedeutet.

3. Salze aus (+)-8-Halogen-6-hydroxy-octansäure und R-(+)-α-Methylbenzylamin, (+)-8-Halogen-6-hydroxy-octansäure und S-(-)-α-Methylbenzylamin, (-)-8-Halogen-6-hydroxy-octansäure und R-(+)-a-Methylbenzylamin oder (-)-8-Halogen-6-hydroxyoctansäure und S-(-)-α-Methylbenzylamin, wobei Halogen Chlor, Brom oder Jod bedeutet.

4. Verfahren zur Herstellung von (+)- und (-)-8-Halogen-6-hydroxy-octansäuren nach Anspruch 1, dadurch gekennzeichnet, daß man ein razemisches Gemisch aus (+)-8-Halogen-6-hydroxy-octansäure und (-)-8-Halogen-6-hydroxy-octansäure oder ein beliebiges Gemisch aus (+)-8-Halogen-6-hydroxy-octansäure und (-)-8-Halogen-6-hydroxy-octansäure mit den Enantiomeren des α-Methylbenzylamins in Lösung umsetzt, die Diastereomerenverbindungen auskristallisieren läßt und die erhaltenen reinen diastereomeren Salze aus (+)-8-Halogen-6-hydroxy-octansäure und α-Methylbenzylamin sowie aus (-)-8-Halogen-6-hydroxy-octansäure und α-Methylbenzylamin durch Umsetzung mit anorganischen oder organischen Säuren bzw. Basen spaltet.

5. Verfahren nach Anspruch 4, dadurch gekennzeichnet, daß man nach der Umsetzung das ausfallende Salz abfiltriert und die Mutterlaugen weiter verarbeitet.

6. Verfahren zur Herstellung von (+)- und (-)-8-Halogen-6-hydroxy-octansäurealkylestern nach Anspruch 2, dadurch gekennzeichnet, daß man die optisch reinen Isomere der 8-Halogen-6-hydroxy-octansäuren in Gegenwart von katalytischen Mengen HCI bei 50 bis 100°C, insbesondere bei 60°C, im entsprechenden Alkohol als Lösungsmittel umsetzt.

7. Verwendung von einer Verbindung nach einem der Ansprüche 1 bis 3 als Zwischenprodukt zur Herstellung von enantiomerenreiner α-Liponsäure sowie enantiomerenreiner Dihydroliponsäure.

## Claims

1. (+)- and (-)-8-halo-6-hydroxyoctanoic acid, where halogen is chlorine, bromine or iodine.

2. Alkyl (+)- and (-)-8-halo-6-hydroxyoctanoates, where halogen is chlorine, bromine or iodine and alkyl is a straight-chain or branched C₁-C₄-alkyl group.

3. Salts of (+)-8-halo-6-hydroxyoctanoic acid and R-(+)-α-methylbenzylamine, (+)-8-halo-6-hydroxyoctanoic acid and S-(-)-α-methylbenzylamine, (-)-8-halo-6-hydroxyoctanoic acid and R-(+)-a-methylbenzylamine or (-)-8-halo-6-hydroxyoctanoic acid and S-(-)-α-methylbenzylamine, where halogen is chlorine, bromine or iodine.

4. Process for the preparation of (+)- and (-)-8-halo-6-hyroxyoctanoic acids according to Claim 1, characterized in that a racemic mixture of (+)-8-halo-6-hydroxyoctanoic acid and (-)-8-halo-6-hydroxyoctanoic acid or any desired mixture of (+)-8-halo-6-hydroxyoctanoic acid and (-)-8-halo-6-hydroxyoctanoic acid is reacted with the enantiomers of α-methylbenzylamine in solution, the diastereomeric compounds are allowed to crystallize out and the pure diastereomeric salts of (+)-8-halo-6-hydroxyoctanoic acid and α-methylbenzylamine and of (-)-8-halo-6-hydroxyoctanoic acid and α-methylbenzylamine obtained are cleaved by reaction with inorganic or organic acids or bases.

5. Process according to Claim 4, characterized in that the precipitating salt is filtered off and the mother liquors are processed further after the reaction.

6. Process for the preparation of alkyl (+)- and (-)-8-halo-6-hydroxyoctanoates according to Claim 2, characterized in that the optically pure isomers of 8-halo-6-hydroxyoctanoic acids are reacted at 50 to 100°C, in particular at 60°C, in the presence of catalytic amounts of HCl, in the corresponding alcohol as a solvent.

7. Use of a compound according to one of Claims 1 to 3 as an intermediate for the preparation of enantiomerically pure α-lipoic acid and enantiomerically pure dihydrolipoic acid.

## Revendications

1. Acide (+) - et (-)-8-halogéno-6-hydroxy-octanoïque, l'halogène signifiant le chlore, le brome ou l'iode.

2. Ester alkylique d'acides (+)- et (-)-8-halogéno-6-hydroxy-octanoïques, l'halogène étant le chlore, le brome ou l'iode et le fragment alkyle représentant un groupe alkyle en C1-C4 à chaîne droite ou ramifié.

3. Sel d'acide (+)-8-halogéno-6-hydroxy-octanoïque et de R-(+)-α-méthylbenzylamine, d'acide (+)-8-halogéno-6-hydroxy-octanoïque et de S-(-)-α-méthylbenzylamine, d'acide (-)-8-halogéno-6-hydroxy-octanoïque et de R-(+)-α-méthylbenzylamine ou d'acide (-)-8-halogéno-6-hydroxyoctanoïque et de S-(-)-α-méthylbenzylamine, l'halogène étant le chlore, le brome ou l'iode.

4. Procédé pour la préparation d'acides (+)- et (-)-8-halogéno-6-hydroxy-octanoïques selon la revendication 1,
caractérisé en ce qu'
on fait réagir en solution un mélange racémique d'acide (+)-8-halogéno-6-hydroxy-octanoïque et d'acide (-)-8-halogéno-6-hydroxy-octanoïque ou un mélange quelconque d'acide (+)-8-halogéno-6-hydroxy-octanoïque et d'acide (-)-8-halogéno-6-hydroxy-octanoïque avec les énantiomères de l'α-méthylbenzylamine, on fait cristalliser les composés diastéréoisomères, et les sels obtenus sous forme de diastéréoisomères purs d'acide (+)-8-halogéno-6-hydroxy-octanoïque et d'α-méthylbenzylamine ainsi que d'acide (-)-8-halogéno-6-hydroxy-octanoïque et d'α-méthylbenzylamine sont coupés par la réaction avec des acides organiques ou minéraux ou des bases organiques ou minérales.

5. Procédé selon la revendication 4,
caractérisé en ce qu'
après la réaction, on sépare par filtration le sel précipité et on poursuit le traitement des liqueurs-mères.

6. Procédé pour la préparation d'esters alkyliques d'acides (+)- et (-)-8-halogéno-6-hydroxy-octanoïques selon la revendication 2,
caractérisé en ce qu'
on fait réagir les isomères optiquement purs des acides 8-halogéno-6-hydroxy-octanoïques en présence de quantités catalytiques de HCl, à 50-100°C, en particulier à 60°C, dans l'alcool approprié en tant que solvant.

7. Utilisation d'un composé selon l'une des revendications 1 à 3,
en tant que produit intermédiaire pour la préparation d'acide α-lipoïque sous forme d'énantiomère pur, ainsi que d'acide dihydrolipoïque sous forme d'énantiomère pur.
